# EUROPÄISCHE PATENTANMELDUNG

(11) **EP 2 422 735 A1**
(43) Veröffentlichungstag der Anmeldung: **29.02.2012**
(21) Anmeldenummer: 10075373.0
(22) Anmeldetag: 27.08.2010
(51) Int. Cl.: A61B 19/00, A61M 1/12

(54) **Implantierbare Blutfördereinrichtung, Manipulationseinrichtung sowie Koppeleinrichtung**

(71) Anmelder: ECP Entwicklungsgesellschaft mbH, 12247 Berlin (DE)
(72) Erfinder: Schumacher, Jörg, 14513 Teltow (DE)
(74) Vertreter: Pfenning, Meinig & Partner GbR

(57) **Zusammenfassung**

Die Erfindung sieht ein implantierbares Bauteil (1) vor, das mittels einer Koppeleinrichtung, die ein erstes Koppelement (23) und ein zweites Koppelelement (20) vorsieht, mit einer Manipuliereinrichtung (20, 21, 22) selbsthaltend verbindbar ist. Damit kann durch die Manipuliereinrichtung, die beispielsweise vor einem therapeutischen Eingriff bereits mit dem implantierbaren Bauteil verbunden und von diesem nach dem chirurgischen Eingriff wieder getrennt werden kann, das implantierbare Bauteil (1) frei positioniert werden. Dies ist insbesondere bei minimal invasiven Eingriffen vorteilhaft.

## Beschreibung

Die Erfindung liegt auf dem Gebiet der Medizintechnik, insbesondere der Implantatsmedizin, und befasst sich im Besonderen mit den Möglichkeiten des Implantierens von Bauteilen mit minimalem Aufwand bei minimalinvasiven Eingriffen.

Die Implantatsmedizin erlaubt das Einsetzen verschiedenster Aggregate und Elemente in einen Patientenkörper zum Ersatz bestimmter Organe und Körperteile in vorübergehender oder dauerhafter Weise oder das Einfügen bestimmter Aggregate zur Unterstützung von Körperfunktionen.

Solche Aggregate können bei weiträumiger Öffnung des Patientenkörpers meist in einfacher Weise an Ort und Stelle gebracht werden, wobei jedoch ein beträchtliches Operationstrauma entsteht. Die Verringerung des Operationstraumas erfordert kleinere Eingriffe, wobei teilweise die Öffnungen zum Einbringen von Bauteilen in den Körper nur wenig größer oder sogar, bei elastischer Aufweitung, kleiner als die einzubringenden Bauteile sind. Entsprechende Aggregate können in Teilen eingebracht und auch innerhalb des Körpers zusammengesetzt werden.

Das minimalinvasive Einbringen erlaubt oft nicht die direkte Handhabung von Bauteilen mit der Hand, so dass zusätzliche Instrumente wie zum Beispiel Zangen verwendet werden. Jedoch ist auch die Verwendung derartiger Haltegeräte nicht immer einfach. Oft sind, insbesondere bei Herzunterstützungssystemen mit einer Vielzahl von Blut führenden Teilen, die zu implantierenden Bauteile möglichst klein gestaltet und auch abgerundet, um im Körper möglichst wenig zu stören. Dies bringt andererseits eine schwierige Handhabbarkeit durch entsprechende Haltegeräte, wie Zangen, mit sich. Auch ein zielgenaues Platzieren oder das Zusammenbauen einzelner Komponenten im Patientenkörper ist schwierig. Dies kann insbesondere bei Herzunterstützungssystemen der Fall sein, wenn Pumpen, Kanülen, Kabel und Antriebskomponenten zusammengesetzt werden müssen.

Aus dem Stand der Technik sind verschiedene Hilfsmittel zum Implantieren von Teilen von Herzunterstützungssystemen in einen Patientenkörper bekannt.

Aus der PCT/US2008/081082 ist eine Vorrichtung mit einem Anker bekannt, der herznah einsetzbar ist und der mit einer Führungsfaser verbunden ist. Auf der Führungsfaser ist ein schlauchartiger Hohlkörper zu dem Anker verschiebbar und kann dort mittels einer Magneteinrichtung gehalten werden.

Aus der WO 2009/029387 A1 ist eine Handhabungseinrichtung für eine Kanüle bekannt, die einen durch das Innere der Kanüle verlaufenden Führungskörper und zum Halten der Kanüle einen kissenartig radial aufweitbaren Haltekörper aufweist. Dieser ist so weit aufweitbar, dass er sich im Inneren der Kanüle verklemmt.

Der vorliegenden Erfindung liegt vor dem Hintergrund des Standes der Technik die Aufgabe zugrunde, ein Blut führendes implantierbares Bauteil zu schaffen, das mit einer entsprechenden Manipulationseinrichtung effektiv zusammenwirkt, um ein zuverlässiges Einsetzen und Positionieren in einen Patientenkörper bei minimal invasiver Operation zu ermöglichen. Weiterer Gegenstand der Lösung ist eine entsprechende Manipulationseinrichtung, eine Koppeleinrichtung zur mechanischen Kopplung eines implantierbaren Bauteils mit einer Manipulationseinrichtung sowie das System, bestehend aus dem Bauteil und der Manipulationseinrichtung, und entsprechende Verfahren zum Verbinden des Bauteils mit einer Manipulationseinrichtung sowie zu deren Trennung.

Die Aufgabe wird durch ein implantierbares Bauteil gemäß der Erfindung, eine Manipulationseinrichtung zur Handhabung eines derartigen Bauteils, eine Koppeleinrichtung zur mechanischen Kopplung eines Bauteils mit einer Manipulationseinrichtung, ein System mit wenigstens einem implantierbaren Bauteil und wenigstens einer Manipulationseinrichtung, durch ein entsprechendes Verfahren zum Verbinden eines implantierbaren Bauteils mit einer Manipulationseinrichtung und durch ein Verfahren zum Trennen eines implantierbaren Bauteils von einer Manipulationseinrichtung gelöst.

Die Erfindung bezieht sich auf ein implantierbares Bauteil, das einen im Betrieb innerhalb eines menschlichen oder tierischen Körpers Blut führenden Kanal begrenzt und das insbesondere einen Teil einer Blutfördereinrichtung bildet, mit einem Koppelelement, das zur mechanischen Ankopplung einer Manipulationseinrichtung eingerichtet ist.

Ein solches Bauteil kann grundsätzlich jedes in einem Patientenkörper verwendbare Bauteil sein, insbesondere ein Teil eines Herzunterstützungssystems, eine Blutpumpe, ein Ventil, eine Kanüle, ein Katheter, entsprechende Verbindungssysteme zwischen diesen Bauteilen sowie eine Medikamentendosierungspumpe.

Viele dieser Bauteile sind, um Läsionen beim Einsetzen oder beim Verbleib in einem Körper zu vermeiden, mit besonders glatten und abgerundeten Oberflächen ausgestattet. Dies macht die Handhabung durch die Hand des Chirurgen oder eine Greifzange schwierig. Aus diesem Grund ist gemäß der Erfindung ein Koppelelement vorgesehen, das zur mechanischen Ankopplung einer Manipulationseinrichtung an das Bauteil eingerichtet ist. Unter einer Manipulationseinrichtung wird in diesem Zusammenhang beispielsweise ein chirurgisches Instrument verstanden, das beispielsweise einen Handgriff am proximalen, dem Operateur zugewandten Ende aufweist, sowie einen Schaft und ein distales Ende, das mit dem Koppelelement des Bauteils verbindbar ist.

Am distalen Ende der Manipulationseinrichtung kann zu diesem Zweck ein weiteres Koppelelement vorgesehen sein, das mit dem an dem implantierbaren Bauteil befestigten oder in dieses integrierten ersten Koppelelement verbindbar ist.

Es wird somit eine mechanische Kopplung geschaffen, die ein zuverlässiges Manipulieren des implantierbaren Bauteils von außerhalb des Körpers mittels der Manipulationseinrichtung sowohl beim ersten Implantieren als auch später gestattet.

Es kann an dem implantierbaren Bauteil auch mehr als ein Koppelelement angeordnet sein, um mehrere Manipulationseinrichtungen oder eine geteilte Manipulationseinrichtung verwenden zu können, die in diesem Fall vereinfacht auch zur Ausrichtung des implantierbaren Bauteils eingesetzt werden kann. Die mehreren Koppelelemente an dem implantierbaren Bauteil sind zu diesem Zweck voneinander an der Oberfläche des implantierbaren Bauteils beabstandet und können auch dazu genutzt werden, abhängig vom Implantationsort den geeignetsten Zugangsweg wählen zu können.

Das Koppelelement an dem Bauteil ist weiterhin zur selbsthaltenden Ankopplung einer Manipulationseinrichtung eingerichtet. Dies bedeutet, dass das implantierbare Bauteil nicht dauerhaft durch Betätigung der Manipulationseinrichtung festgehalten werden muss, wie das beispielsweise bei verschiedenen chirurgischen Zangen der Fall ist. Die Kopplung zwischen dem Bauteil und der Manipulationseinrichtung ist vorteilhaft selbsthaltend ausgestaltet, so dass die Manipulationseinrichtung auch zeitweise vom Operateur außer Acht gelassen werden kann, ohne dass die Kopplung aufgelöst wird.

Vorteilhaft sind die Koppelelemente so ausgestaltet, dass entsprechende Koppelkräfte ausschließlich zwischen den Koppelelementen entwickelt werden, beispielsweise durch elastische Verformung eines der Koppelelemente, ohne dass hierzu Kräfte über den Schaft der Manipulationseinrichtung übertragen werden.

Hierdurch kann der Operateur sich nacheinander auf verschiedene Manipulationseinrichtungen konzentrieren, und es ist sichergestellt, dass die relative Position einer Manipulationseinrichtung zu dem implantierbaren Bauteil stabil und unverrückbar festgelegt ist.

Das Koppelelement kann in das Bauteil integriert oder mit diesem fest verbunden sein. Dabei sollte vorteilhaft die Kopplung zwischen dem an dem Bauteil befestigten oder in dieses integrierten Koppelelement und der Manipulationseinrichtung besonders einfach auch wieder auflösbar sein, damit die Manipulationseinrichtung nach Positionierung und Montage des implantierbaren Bauteils ohne weiteres aus dem Patientenkörper entfernbar ist.

Das Koppelelement selbst kann dabei beispielsweise als ein Innengewinde in dem implantierbaren Bauteil, vorzugsweise innerhalb einer Bohrung, ausgebildet sein, in das ein weiteres Koppelelement in Form eines Gewindezapfens einschraubbar ist.

Das Koppelelement kann ebenso als Bohrung oder als eine Gruppe von Bohrungen in dem Bauelement ausgeführt sein, in die Bolzen eines weiteren Koppelelementes einführbar sind. Entsprechende Bolzen können beispielsweise aus Metall, jedoch auch aus elastischen Werkstoffen wie beispielsweise Gummi oder auch Federdraht bestehen, die kraftschlüssig in die entsprechenden Bohrungen eingeführt werden können. Ein Koppelelement kann typisch auch als eine Nut, beispielsweise mit einer Hinterschneidung, beispielsweise als Schwalbenschwanznut, ausgeführt sein, in die ein entsprechend komplementär ausgeführter Körper eines weiteren Koppelelementes eingeschoben werden kann.

Auch Bohrungen bzw. Sacklöcher oder andere Öffnungen mit oder ohne Hinterschneidungen können ein Koppelelement bilden, wobei das jeweils komplementäre Koppelelement dann beispielsweise einen verformbaren und/oder bewegbaren Körper mit Vorsprüngen und/oder Rastelementen aufweisen kann, die eine Kopplung bewirken und die entweder mittels eines Betätigungsorgans entrastet werden können oder die bei Aufbieten einer Zugkraft und Überschreiten einer bestimmten Auslösekraft verformbar sind, um die Koppelelemente zu trennen. Dazu kann beispielsweise eines der Koppelelemente wenigstens teilweise aus einem Elastomer oder einer Feder bestehen.

Beispielsweise kann die Manipulationseinrichtung ein entsprechendes Koppelelement enthalten und zudem einen hohlen Schaft, der mit einem Griff gekoppelt ist. Innerhalb des Schaftes kann das entsprechende Betätigungsorgan für Rastnasen oder zum Lösen einer Verschraubung im Bereich der Kopplung zwischen den Koppelelementen vorgesehen sein, und das Betätigungsorgan kann im Bereich des Griffs der Manipulationseinrichtung betätigbar sein.

Es ist auch denkbar, ein Koppelelement als einen Konus auszubilden, der in dem Bauteil, beispielsweise einer Wand des Bauteils, integriert ist und in den ein weiterer Konus einschiebbar ist, der ein zweites Koppelelement bildet, das mit einer Manipulationseinrichtung verbunden ist. Die Konusflächen können beispielsweise durch eine Presspassung, jedoch auch mittels Klebstoff oder durch einen vakuumdichten Abschluss miteinander gekoppelt sein. Auch Schrägkonusklemmen können für eine solche Verbindung verwendet werden.

Es ist außerdem denkbar, dass als Koppelelement eine oder mehrere Schlüsselflächen an dem implantierbaren Bauteil dienen, die das formschlüssige Angreifen eines entsprechenden Koppelelementes der Manipulationseinrichtung erlauben.

Es kann vorteilhaft auch vorgesehen sein, dass das entsprechende bauteilseitige Koppelelement an dem implantierbaren Bauteil befestigt, beispielsweise an dieses angeklebt, angeschweißt oder angelötet oder auch einteilig mit diesem ausgebildet ist. Dies kann beispielsweise dadurch ausgeführt sein, dass eine Koppelplatte mit einer Gewindebohrung oder eine Öse oder ein massiver Körper, der seinerseits eine Bohrung, eine Nut, Schlüsselflächen oder Konusflächen aufweisen kann, an dem implantierbaren Bauteil befestigt ist.

Das Koppelelement kann beispielsweise auch als eine glatte, oberflächengeglättete Fläche ausgebildet sein, auf die ein als zweites Koppelelement dienender Saugnapf aufsetzbar ist. Der Saugnapf kann durch den Schaft der Manipulationseinrichtung, mit der er verbunden ist, beispielsweise gezielt be- und entlüftbar sein, um die Kopplung zwischen den Koppelelementen gezielt herzustellen und zu lösen.

Auch die Verbindung zweier glatter Koppelflächen von zwei Koppelelementen mittels eines Klebstoffs ist denkbar, wobei der Klebstoff zum Lösen der Verbindung gezielt auflösbar oder versprödbar ist. Der Klebstoff kann beispielsweise als Thermoplast ausgebildet sein, der kurzzeitig verflüssigbar ist, um die Koppelelemente voneinander zu lösen, oder als härtbares Harz, das durch Strahlungsvernetzung, beispielsweise durch UV-Licht, versprödbar ist, so dass die Klebeverbindung leicht gebrochen werden kann.

Es ist auch denkbar, das implantierbare Bauteil bei der Herstellung durch Verguss mit einem Koppelelement der Manipulationseinrichtung einstückig zu verbinden, so dass das implantierbare Bauteil, das mit diesem verbundene Koppelelement, das Koppelelement der Manipulationseinrichtung und wenigstens ein Teil der Manipulationseinrichtung selbst oder die gesamte Manipulationseinrichtung einstückig beispielsweise in einem Spritzgussverfahren herstellbar sind.

Es ist dann eine Sollbruchstelle zwischen den Koppelelementen des implantierbaren Bauteils und der Manipulationseinrichtung vorgesehen, um nach dem Platzieren des implantierbaren Bauteils die Manipulationseinrichtung durch Brechen der Kopplung zwischen den Koppelelementen entfernen zu können.

Als bauteilseitiges Koppelelement kann beispielsweise auch ein Magnet oder ein ferromagnetisches Werkstück vorgesehen sein, das an der Außenseite oder im Inneren des Bauteils angeordnet ist und das mit einem Magneten oder einem ferromagnetischen Teil der Manipulationseinrichtung zur Kopplung zusammenwirkt. Als Magnet im Bauteil kann beispielsweise auch ein Teil eines Motors oder Rotors wirken. An der Außenseite des Bauteils kann zur Ankopplung der Manipulationseinrichtung eine Passfläche vorgesehen sein. Einer der Magneten der Koppeleinrichtung kann als schaltbarer Elektromagnet ausgebildet sein. Eine entsprechende Passfläche kann beispielsweise, wenn sie einen Teil des bauteilseitigen Koppelelements bildet, als mechanische Fangeinrichtung für das zweite, der Manipulationseinrichtung zugeordnete Koppelelement ausgebildet sein und eine Ausnehmung aufweisen, die Einführschrägen oder ähnliche Einführhilfen für das zweite Koppelelement aufweist. Eine Einführschräge kann beispielsweise in einen Einführkonus, eine Hohlkalotte oder eine andere sich zum Bauteilinneren hin verjüngende Ausnehmung integriert sein. Das zweite Koppelelement kann gegebenenfalls eine komplementäre geometrische Form aufweisen.

Das Bauteil kann, insbesondere wenn das erste Koppelelement als Passfläche, besonders mit Einführschräge, ausgebildet ist und insbesondere dann, wenn die Passfläche selbst sich nicht in unmittelbarer Nähe eines ferromagnetischen Körpers des Bauteils befindet, jedoch auch in allen anderen hier beschriebenen Fällen, derart ausgebildet sein, dass die Kontur der Passfläche durch bildgebende Verfahren, beispielsweise Röntgen oder Ultraschall, darstellbar ist. Dies ist z. B. durch einen metallischen Oberflächenbelag des Bauteils im Bereich der Passfläche möglich oder durch einen Markerkörper, der eine höhere Dichte aufweist als die Wand des Bauteils und der in unmittelbarer Nähe der Passfläche oder mit erkennbarem geometrischem Bezug zur Passfläche in das Bauteil integriert ist. Damit ist bei einer nach der ersten Implantation gegebenenfalls notwendig werdenden weiteren Intervention eine Kopplung zwischen einer Manipulationseinrichtung mit einem ferromagnetischen Koppelelement und dem Bauteil leichter auch ohne Sichtkontakt herstellbar.

Als Koppelelement kann auch an einer Außenfläche des Bauteils eine Öse vorgesehen sein, durch die ein Faden geführt wird, der als Koppelelement der Manipulationseinrichtung dient. Eine solche Ausführung ist insbesondere dann von Interesse, wenn ein Teil eines längeren flexiblen Bauteils, wie beispielsweise einer schlauchartigen Kanüle, von einem Eintrittsort zu einem Zielort gezogen werden soll. Die Kopplung kann dann durch Trennen des Fadens leicht getrennt werden.

Grundsätzlich sind bei den oben beschriebenen komplementären Koppelelementen die Zuordnungen zu dem Bauteil einerseits und der Manipulationseinrichtung andererseits austauschbar.

Eine erfindungsgemäße Koppeleinrichtung sieht entsprechend ein erstes Koppelelement vor, das dem implantierbaren Bauteil zugeordnet ist, sowie ein zweites Koppelelement, das der Manipulationseinrichtung zugeordnet ist, wobei die Koppelelemente kraftschlüssig und/oder formschlüssig und/oder stoffschlüssig miteinander verbindbar oder verbunden sind.

Die Koppeleinrichtung ist dabei vorteilhaft so eingerichtet, dass ohne äußere Einwirkung die Kopplung stabil gehalten wird.

Besteht die Kopplung aus einer stoffschlüssigen Verbindung zwischen dem ersten Bauteil und dem zweiten Bauteil, so kann diese durch Brechen, Zerschneiden oder Änderung der mechanischen Stoffeigenschaften wenigstens eines Teils der Koppeleinrichtung getrennt werden. Unter der Änderung der mechanischen Stoffeigenschaften wird beispielsweise eine Versprödung durch Strahlungs- oder Wärmeeinwirkung oder ein Schmelzen eines Teils der Kopplung oder ein Verdampfen, beispielsweise durch Lasereinwirkung, verstanden.

Die Erfindung bezieht sich außer auf ein implantierbares Bauteil, eine Manipulationseinrichtung sowie eine Koppeleinrichtung auch auf ein entsprechendes System mit einem oder mehreren Bauteilen und einer oder mehreren Manipulationseinrichtungen, die entsprechend eingerichtet sind, um mittels der Koppelelemente einfach selbsthaltend koppelbar zu sein. Die entsprechenden Kopplungen müssen innerhalb des Patientenkörpers auch einfach und schonend wieder gelöst werden können.

Die Erfindung bezieht sich weiter auch auf ein Verfahren zum Verbinden eines implantierbaren Bauteils mit einer Manipulationseinrichtung, wobei das Bauteil vor dem Einbringen in den Patientenkörper mittels einer selbsthaltenden, lösbaren Koppeleinrichtung mechanisch mit der Manipulationseinrichtung verbunden wird.

Dies kann beispielsweise bereits bei der Herstellung des implantierbaren Bauteils durch Integration der Koppeleinrichtung geschehen, indem beispielsweise das Bauteil und wenigstens ein Teil der Manipulationseinrichtung mitsamt der Koppeleinrichtung einstückig im Spritzgießverfahren hergestellt oder durch ein Fügeverfahren miteinander verbunden werden.

Das implantierbare Bauteil kann mit der Manipulationseinrichtung auch lösbar verschraubt, verklemmt, verklebt oder verrastet werden, wobei jeweils eines der Koppelelemente mit dem anderen Koppelelement komplementär zusammenwirkt und wobei beispielsweise eine Gewindebohrung in dem Bauteil und ein entsprechender Gewindebolzen an der Manipulationseinrichtung angeordnet ist und wobei jedes der Koppelelemente auch jeweils vertauschbar an dem jeweils anderen Teil vorgesehen sein kann. Ist daher beispielsweise an dem implantierbaren Bauteil eine Öse vorgesehen und an der Manipulationseinrichtung ein entsprechender Haken, so kann der Haken auch an dem implantierbaren Bauteil und die Öse an der Manipulationseinrichtung vorgesehen sein. Dies gilt auch für alle anderen Ausführungsformen der Koppelelemente.

Sind vor dem Beginn eines chirurgischen Eingriffs das implantierbare Bauteil und die Manipulationseinrichtungen bereits miteinander verbunden, so erleichtert und beschleunigt dies den chirurgischen Eingriff.

Die Trennung des implantierbaren Bauteils von der Manipulationseinrichtung kann typischerweise erst nach Ende der Implantation erfolgen, beispielsweise durch mechanisches Brechen der Koppeleinrichtung oder durch andere Trennung der Koppeleinrichtung.

Die Koppeleinrichtung kann vor der Trennung beispielsweise mechanisch geschwächt werden, z. B. durch Kühlung, Aufheizung, Bestrahlung, Biegen oder Knicken der Koppeleinrichtung.

Wenn das Verfahren zum Trennen des implantierbaren Bauteils von der Manipulationseinrichtung auf eine Weise geschieht, die kein Verschieben oder Bewegen der Manipulationseinrichtung gegenüber dem Bauteil vorsieht, dann hat dies den Vorteil, dass die Positionierung des Bauteils nach Beendigung des chirurgischen Eingriffs nicht wieder geändert werden muss. Eine entsprechende Auflösung der Koppeleinrichtung kann beispielsweise durch Zerschneiden oder chemisches Auflösen, Zersetzen, Verflüssigen oder Verdampfen eines Teils der Koppeleinrichtung geschehen.

Im Folgenden wird die Erfindung anhand eines Ausführungsbeispiels in einer Zeichnung gezeigt und nachfolgend beschrieben. Dabei zeigt
- Fig. 1: die Darstellung eines implantierbaren Bauteils und zweier Manipulationseinrichtungen in einer dreidimensionalen Ansicht,
- Fig. 2: schematisch im Schnitt eine Manipulationseinrichtung, einen Abschnitt eines implantierbaren Bauteils sowie eine Koppeleinrichtung,
- Fig. 3: die Ansicht einer Koppeleinrichtung,
- Fig. 4: die Koppeleinrichtung aus Fig. 3 in einer Draufsicht,
- Fig. 5: eine weitere Koppeleinrichtung,
- Fig. 6: eine Koppeleinrichtung, einen Abschnitt eines Bauteils sowie eine Manipulationseinrichtung,
- Fig. 7: einen Abschnitt eines Bauteils sowie eine Koppeleinrichtung,
- Fig. 8: einen Abschnitt eines implantierbaren Bauteils,
- Fig. 9: einen Abschnitt eines implantierbaren Bauteils mit einer Koppeleinrichtung,
- Fig. 10: eine alternative Koppeleinrichtung zu der in Fig. 9 gezeigten,
- Fig. 11: einen Abschnitt eines Bauteils mit einer Koppeleinrichtung,
- Fig. 12: einen Teil eines implantierbaren Bauteils in einem Schnitt mit einer Kopplungseinrichtung,
- Fig. 13: eine weitere Alternative einer Kopplungseinrichtung mit einem Abschnitt eines implantierbaren Bauteils sowie
- Fig. 14: eine Alternative einer Kopplungseinrichtung mit einem aufpumpbaren Koppelelement.

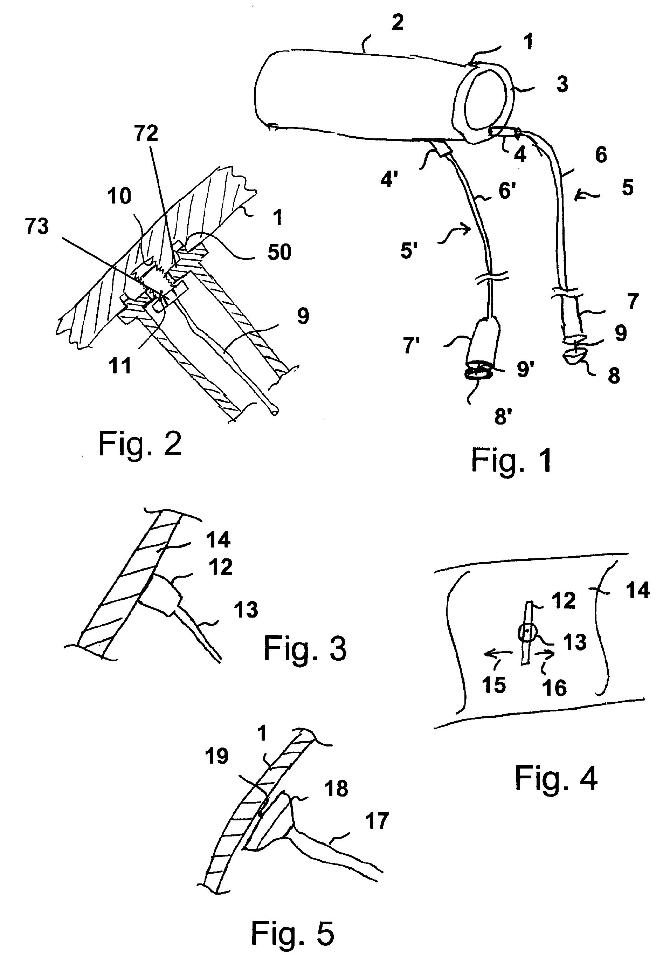

Fig. 1 zeigt schematisch und stilisiert ein implantierbares Bauteil 1, das beispielsweise eine Pumpe zum Fördern von Blut als Teil eines Herzunterstützungssystems darstellt, mit einer zylindrischen Au-βenwand 2 eines Pumpengehäuses sowie einem in dem Hohlzylinder gelagerten Axialrotor, der im Einzelnen nicht dargestellt ist.

An der Stirnseite 3 des Bauteils 1 ist ein Koppelelement 4 einer Manipulationseinrichtung 5 befestigt und mit einem nicht näher dargestellten Koppelelement verbunden, das in das Bauteil 1 integriert ist.

Das Koppelelement 4 ist mit einem Schaft 6 der Manipulationseinrichtung 5 verbunden, die auf ihrem proximalen Ende mit einem Griff 7 verbunden ist. An dem Griff 7 ist zudem ein Knauf 8 vorgesehen, der beispielsweise mit einer Seele 9 verbunden sein kann, die in einem Hohlraum des Schaftes 6 bis zu dem Koppelelement 4 verläuft.

Der Knauf 8 kann gegenüber dem Griff 7 bewegt, beispielsweise verschoben und/oder gedreht werden, um die Seele 9 gegenüber dem Schaft 6 zu bewegen, damit das Koppelelement 4 zu betätigen und beispielsweise die Manipulationseinrichtung 5 von dem Bauteil 1 zu lösen.

Eine weitere Manipulationseinrichtung 5' ist über ein weiteres Koppelelement 4' mit dem Bauteil 1 an einer anderen Stelle verbunden. Die weitere Manipulationseinrichtung 5' weist ebenfalls einen Griff 7' und einen Knauf 8' auf. Durch beide Manipulationseinrichtungen 5, 5' gemeinsam, die an dem Bauteil 1 an voneinander beabstandeten Stellen befestigt sind, kann das Bauteil 1 nicht nur verschoben, sondern auch gedreht werden.

Auch die zweite Manipulationseinrichtung 5' kann innerhalb eines hohlen Schaftes 6' eine Seele 9' aufweisen, die mittels des Knaufs 8' bewegbar ist.

Fig. 2 zeigt eine beispielhafte Implementierung einer Koppeleinrichtung 10 an dem Bauteil 1 mit einem Sackloch, das ein Innengewinde aufweist, in das ein Gewindebolzen 11 eingeschraubt ist. Der Gewindebolzen 11 als Koppelelement der Manipulationseinrichtung ist mit einer Seele 9 verbunden, die durch einen hohlen Schaft 6 verläuft und in diesem gedreht werden kann, um den Gewindebolzen 11 in die Gewindebohrung 10 hinein oder aus dieser herauszudrehen. Damit kann der Schaft 6 an dem Bauteil 1, das in Fig. 2 nur abschnittsweise dargestellt ist, befestigt und von diesem wieder gelöst werden.

Grundsätzlich kann ein Koppelelement durch eine Ausnehmung, insbesondere eine Bohrung oder Nut, mit einer Hinterschneidung oder ohne Hinterschneidung gebildet sein.

Durch zusätzliche Zapfen am Schaft, die in korrespondierende Bohrungen des Implantats eingreifen, wird verhindert, dass sich das Implantat beim Lösen der Schraubverbindung mitdreht. Dies gewährleistet darüber hinaus, dass das Implantat auch drehend manipuliert werden kann.

Es kann auch innen in dem Schaft 6 ein Flansch 72 vorgesehen sein, der mittels des Gewindebolzens 11 und eines Kopfes 73 des Gewindebolzens durch Eindrehen in die Gewindebohrung 10 an dem Bauteil 1 verschraubt werden kann.

Fig. 3 zeigt schematisch ein Koppelelement 12 einer Manipulationseinrichtung in Form eines flachen, plattenförmigen Thermoplastendteils, an das ein Schaft 13 angesetzt ist, der einen Teil einer Manipulationseinrichtung bildet. Das plattenförmige Teil 12 ist mit dem beispielsweise eben ausgebildeten und ein bauteilseitiges Koppelelement bildenden Wandstück 14 verbunden, beispielsweise an dieses angeschweißt, angeklebt oder mit diesem einstückig als Spritzgussteil hergestellt. Damit kann mittels des Schaftes 13 das Bauteil 1 manipuliert werden, bis das Koppelelement 12 durch Abknicken der Platte 12 an dem Bauteil abgebrochen 1 wird.

Zur besseren Übersichtlichkeit ist diese Konfiguration in der Fig. 4 in einer Draufsicht dargestellt, wobei die Platte 12 und der Schaft 13 erkennbar sind. Die Platte 12 kann durch Schwenken in Richtung der Pfeile 15, 16 abgeknickt und entfernt werden.

Der Ansatz des plattenförmigen Stücks 12 in der Wand 14 des Bauteils 1 bildet das dort integrierte Koppelelement, mit dem der abknickbare Teil der Platte 12 als Koppelelement der Manipulationseinrichtung verbunden ist.

Gemäß der Fig. 5 ist ein Schaft 17 mit einem glockenförmigen Koppelelement 18 verbunden, das an dem implantierbaren Bauteil 1 mittels eines kurzen Drahtes 19 verbunden ist. Der Draht 19 sorgt dafür, dass die Glocke 18 auf dem Bauteil 1 aufsitzt und damit das Bauteil 1 mittels des Schaftes 17 bewegt werden kann.

Durch eine Drehung des Schaftes 17 an seine Längsachse oder durch ein Kippen des Glockenteils mit grö-βerer Kraftanwendung kann der Draht 19 abgedreht oder abgerissen werden um die Manipulationseinrichtung 17, 18 von dem Bauteil 1 zu entfernen. Die Koppelelemente 18, 19 sorgen solange die Verbindung besteht für eine gute Handhabbarkeit des Bauteils 1 über die Manipulationseinrichtung.

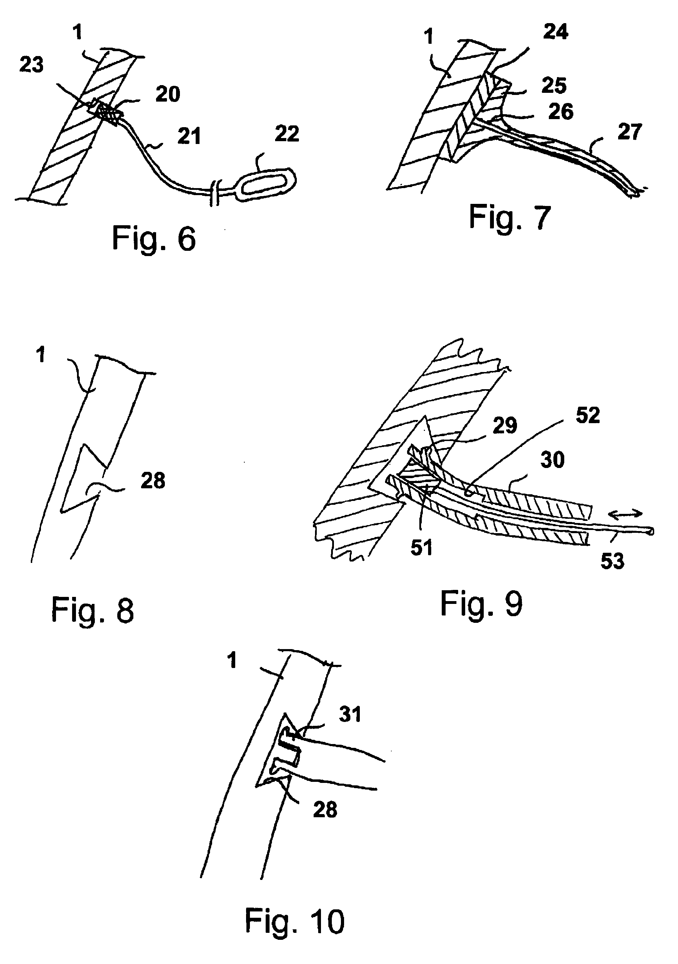

In der Fig. 6 ist eine Koppeleinrichtung mit einem aus einem Elastomer bestehenden Koppelelement 20 gezeigt, das mittels eines Schaftes 21 mit einem Griff 22 einer Manipulationseinrichtung verbunden ist. Das Element 20 ist in einer Bohrung 23 als Koppelelement eines implementierbaren Bauteils 1 kraftschlüssig festgelegt. Beispielsweise kann das Element 20 erhitzt und in die Bohrung 23 eingepresst werden um eine Verbindung der Kopplungseinrichtung 20, 23 herzustellen. Nachher kann die Manipulationseinrichtung 20, 21, 22 durch Ziehen an dem Schaft 21 von dem Bauteil 1 entfernt werden. Es ist auch denkbar, das Koppelelement 20 vorher zu erwärmen oder stark abzukühlen um es schrumpfen zu lassen.

In der Fig. 7 ist eine Koppeleinrichtung gezeigt mit einem ersten Bauteil 1, auf das ein Koppelelement in Form einer Platte 24 aufgesetzt, beispielsweise angeschweißt, gelötet oder geklebt ist. Die Platte 24 kann auch als integraler Bestandteil des Bauteils 1 ausgeführt sein.

Auf der glatten Oberfläche des Koppelelements 24 ist ein Saugnapf 25 aufgesetzt, der beispielsweise aus einem Elastomer bestehen kann, jedoch auch aus einer harten hochpolierten Platte, beispielsweise aus Stahl. Die beiden Platten 24, 25 haften als Koppelelemente bei ausreichender Oberflächengüte saugend aneinander. Dies ist insbesondere dann der Fall, wenn eins der Bauteile als elastomerer Saugnapf ausgebildet ist.

Innerhalb des zweiten Koppelelements 25 kann ein Belüftungskanal 26 vorgesehen sein, der durch den Schaft 27 verläuft und vom proximalen Ende der Manipulationseinrichtung 25, 26, 27 her belüftet oder evakuiert werden kann, um die Kopplungseinrichtung 24, 25 zu koppeln oder zu lösen.

In der Fig. 8 ist schematisch ein Abschnitt eines implantierbaren Bauteil 51 mit einer schwalbenschwanzartigen Nut 28 bzw. einer Bohrung mit Hinterschneidungen dargestellt.

Handelt es sich um eine radial symmetrische Bohrung mit Hinterschneidungen, so kann gemäß Fig. 9 in diese ein tulpenartiges Koppelelement 29 mit nach außen weisenden Rastnasen eingesetzt werden, die in den Hinterschneidungen der Bohrung 28 elastisch einrasten. Das Koppelelement 29 ist mit einem Schaft 30 einer Manipulationseinrichtung verbunden und kann durch ausreichenden Zug an diesem Schaft 30 auch aus der Bohrung 28 nur dann herausgezogen werden, wenn ein Blockierelement 51 in Form eines Zylinders innerhalb einer Ausnehmung 52 in der Manipuliervorrichtung mittels Bewegung der Seele 53 axial von den Rastnasen 29 beabstandet wird und damit eine Einwärtsbewegung der Rastnasen freigibt. Der Schaft 30 ist hohl ausgebildet, um die Seele 53 aufzunehmen.

In der Fig. 10 ist eine andere Konstellation eines Koppelements 31 gezeigt, das ebenso in einer Bohrung des implantierbaren Bauteils 1 mit Hinterschneidungen angeordnet ist und das im Wesentlichen zylindrisch mit nach außen weisenden Rastnasen von elastisch biegbaren Endbereichen von End-Fingern des Koppelelements ausgebildet ist. Auch das Koppelelement 31 sitzt fest in der Bohrung 28, ist jedoch durch Kraftanwendungen aus dieser herausziehbar oder durch Einziehen der Rastnasen des Koppelelements 31.

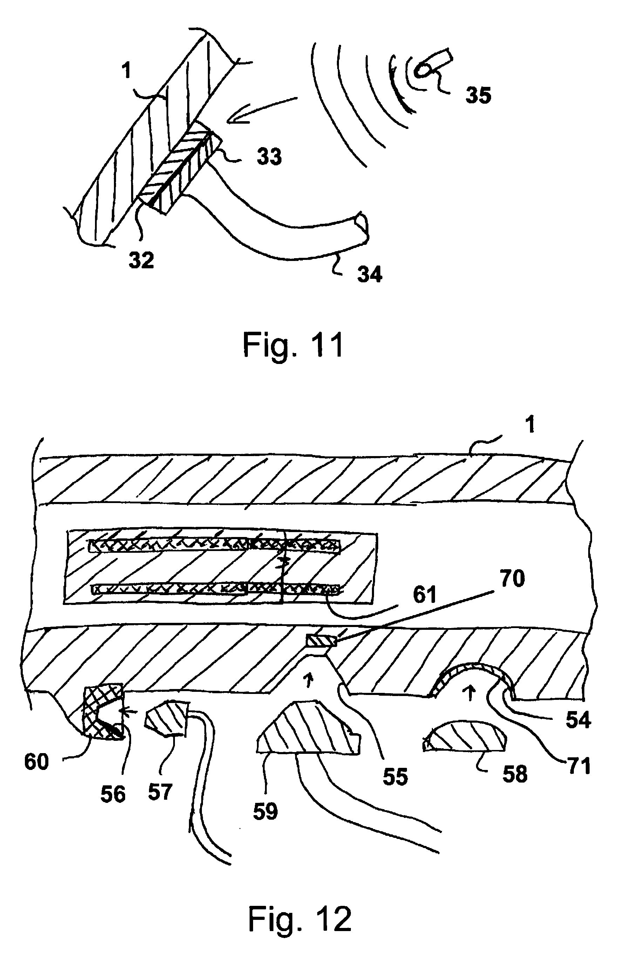

Die Fig. 11 zeigt eine Kopplungsvorrichtung mit einem ersten Koppelelement 32 und einem zweiten Koppelelement 33, die jeweils plattenförmig ausgebildet sind. Das erste Koppelelement 32 ist an einem Abschnitt eines implantierbaren Bauelements 1 durch Kleben, Schweißen oder sonstige Fügetechnik befestigt und weist eine ebene glatte Oberfläche an der dem Bauteil 1 abgewandten Seite auf. Auf die Oberfläche ist das zweite Koppelement 33 aufgelegt und dort beispielsweise mittels eines Klebstoffs, z.B. eines Epoxidharzes oder eines anderen härtbaren Harzes, befestigt.

Dadurch sind die Koppelemente miteinander verbunden und der Schaft 34 einer Manipulationseinrichtung ist über diese Kopplungsvorrichtung mit dem Bauteil 1 fest und selbsthaltend verbunden.

Die Verbindung kann beispielsweise vor Einbringen des Bauteils 1 in einen Patientenkörper dauerhaft hergestellt sein.

Zum Auflösen der Kopplungsverbindung kann beispielsweise mittels einer UV-Strahlungsquelle 35 das Epoxidharz zwischen den Koppelelementen 32, 33 so weit nachgehärtet werden, dass dies versprödet, so dass die Koppelemente 32, 33 leicht durch Abbrechen voneinander getrennt werden können.

Es kann allerdings auch vorgesehen sein, dass die Koppelemente 32, 33 durch einen thermoplastischen Kleber miteinander verbunden sind, der durch Bestrahlung, beispielsweise mit einer Wärmequelle, verflüssigbar ist, so dass auf diese Weise die Kopplung auflösbar ist und die Manipulationseinrichtung durch Trennen des zweiten Koppelements 33 von dem ersten Koppelelement 32 entfernbar ist. Auch Ultraschallimpulse oder Laserstrahlen können zum Auflösen der Kopplung eingesetzt werden.

In der Fig. 12 ist eine Konstellation dargestellt, bei der ein hohlzylindrisches Bauteil 1 als Koppelelement beispielhaft eine kalottenförmige Passfläche 54 und zwei konusförmige Passflächen 55, 56 aufweist. In die Passfläche 56 kann der komplementäre Konus 57 parallel zur Längsachse des zylindrischen Bauteils 1 einlaufen, in die übrigen Passflächen können sich die komplementären Körper 58, 59 radial hineinbewegen. Die Körper 57, 58, 59 stellen jeweils Koppelelemente einer Manipulationseinrichtung dar und können als ferromagnetische Körper, gegebenenfalls als Magnete und auch als schaltbare Elektromagnete ausgebildet sein.

Die Passflächen 54, 55, 56 können jeweils von ferromagnetischem und/oder magnetisiertem Material des Bauteils 1 umgeben sein, wie am Beispiel der Passfläche 56 durch Schraffur des magnetischen Bereichs 60 gezeigt.

Es kann auch vorgesehen sein, dass die magnetische Wirkung von magnetisch aktiven Teilen 61 im Inneren des Bauteils 1, beispielsweise von Teilen eines Pumpenantriebs oder eines magnetischen Ventils, zur Kopplung ausgenutzt wird.

Um eine Kopplung der Manipulationseinrichtung mit dem Bauteil mittels bildgebender Verfahren zu erleichtern, kann ein in die Wand des Bauteils integrierter Markerkörper 70 oder ein Oberflächenbelag 71 einer Passfläche 54 aus einem Material hoher Dichte, insbesondere Metall, vorzugsweise einem Edelmetall, Chrom oder Chirurgenstahl, vorgesehen sein.

Zum Lösen einer solchen magnetischen Kopplung kann entweder ein entsprechender Elektromagnet abgeschaltet oder die Manipulationsvorrichtung ruckweise bewegt werden. Es kann auch eine Entmagnetisierung der Magnete mittels eines Wechselfeldes erfolgen. Es kann aber auch durch Drehen des einen Magneten gegenüber dem anderen eine andere Ausrichtung der magnetischen Pole gegeneinander erreicht werden, so dass durch die auftretenden magnetischen Abstoßungskräfte ein vereinfachtes Lösen ermöglicht wird.

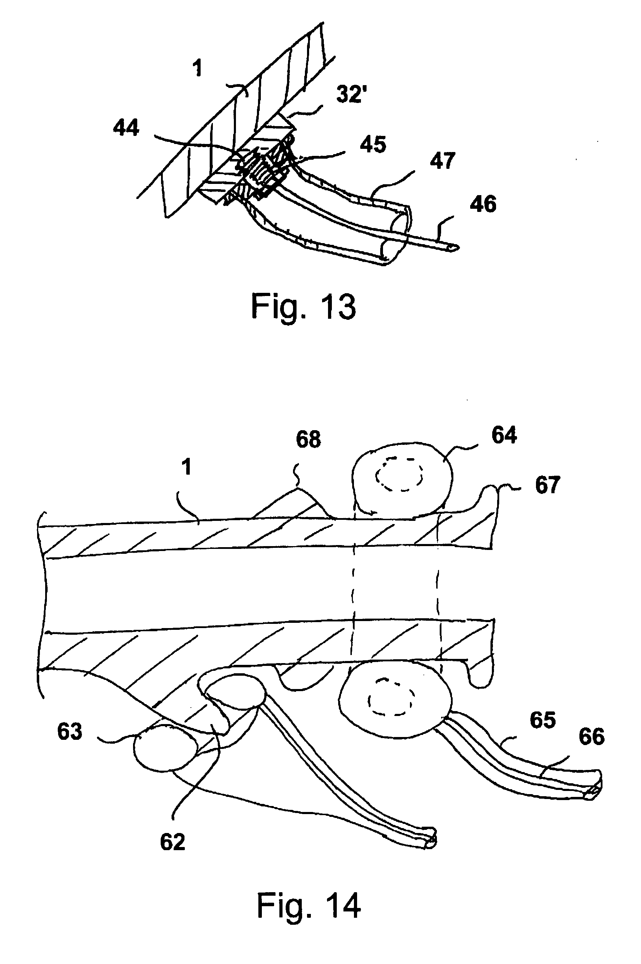

In der Fig. 13 ist eine Kopplungsvorrichtung dargestellt mit einer Platte 32', die an ein implantierbares Bauteil 1 angefügt ist und die eine Sackbohrung 44 mit einem Innengewinde aufweist. Die Platte 32' mit der Sackbohrung 44 stellt dabei ein erstes Koppelelement des Bauelements 1 dar. Mit diesem ersten Koppelelement ist ein zweites Koppelelement verbindbar, das einen Gewindebolzen 45 aufweist, der mit einer Seele 46 verbunden ist. Die Seele 46 verläuft im Hohlraum eines Schaftes 47, der einen Teil einer Manipuliereinrichtung darstellt und proximal mit einem in Fig. 13 nicht dargestellten Griff verbunden ist. Mittels des Gewindebolzens 44 werden der Schaft 47 und die Seele 46 mit dem Koppelelement 32' des implantierbaren Bauteils 1 verbunden.

Wird über einen Knauf am Griff der Manipuliereinrichtung die Seele 46 gegenüber dem Schaft 47 gedreht, so kann hierdurch der Gewindebolzen 45 in die Platte 32' hinein und aus dieser bzw. der Gewindebohrung 44 herausgedreht werden, um, je nach der Situation, die Kopplung herzustellen oder zu trennen.

Im Zustand hergestellter Kopplung ist die Kopplungsvorrichtung selbsthaltend, d. h., an dem nicht dargestellten Griff der Manipulationseinrichtung kann das erste Bauteil 1 ohne weiteres verschoben und wunschgemäß positioniert bzw. gedreht werden.

Es kann auch wahlweise der Gewindebolzen 47 durch eine mit der Seele 46 verbundene Mutter mit Innengewinde und die Gewindebohrung 44 durch einen entsprechenden Gewindebolzen ersetzt werden.

Als weiteres Ausführungsbeispiel kann auch das implantierbare Bauteil eine Schlüsselfläche, beispielsweise eine Sechskantfläche, als Koppelelement aufweisen, an der ein komplementärer Schlüssel als zweites Koppelelement angreifen kann. Wird diese Kombination in Presspassung ausgeführt, so ist die Verbindung selbsthaltend.

Vorteilhaft ist bei den beschriebenen Koppeleinrichtungen vorgesehen, sie am Implantat an der kein Blut führenden Fläche auszubilden. Dadurch kann jede Kontamination bzw. Beschädigung des Blut führenden Kanals verhindert werden. Außerdem werden gegebenenfalls vorgesehene Ausnehmungen, Bohrungen und dergleichen nicht an dem Blut führenden Kanal vorgesehen, so dass Strömungstotgebiete vermieden werden.

Als Koppeleinrichtung kann auch, wie in Fig. 14 gezeigt, eine Kombination aus einem aufpumpbaren Hohlkörper, insbesondere einem Kissen einerseits und einer Greiffläche andererseits, beispielsweise bauteilseitig vorgesehen sein.

Beispielsweise kann ein über den Schaft einer Manipulationseinrichtung aufpumpbares Kissen in einen Hohlraum eines Bauteils eingeführt und dort aufgepumpt werden. Dazu kann der entsprechende Hohlraum eine Hinterschneidung aufweisen. Der aufpumpbare Hohlkörper kann auch als Torus 63, 64 ausgebildet und über das Bauteil 1 oder einen Teil 62 des Bauteils geschoben und dort aufgepumpt werden.

Sobald das Implantat platziert und ausgerichtet ist, kann der Druck in dem Hohlkörper abgesenkt und damit die Koppelvorrichtung getrennt werden.

Der Hohlkörper kann zum besseren Haften an dem Bauteil sowie zur Schonung der Bauteilflächen mit einem Elastomer beschichtet sein.

Zum Aufpumpen und zur Druckabsenkung des Hohlkörpers ist jeweils im Schaft 65 der Manipulationseinrichtung ein Fluidkanal 66 vorgesehen. An dem Bauteil 1 können Wülste 67, 68 zum Halten des Hohlkörpers vorgesehen sein, die somit entsprechende Passflächen als bauteilseitiges Koppelelement bilden.

Durch die verschiedenen dargestellten Varianten einer selbsthaltenden Kopplungsvorrichtung zwischen einem implantierbaren Bauteil und einer Manipulationseinrichtung kann das Bauteil ohne besonderen Aufwand mittels der Manipuliereinrichtung durch einen Operateur einfach positioniert und ausgerichtet werden, ohne dass die Hand des Operateurs direkt in das Operationsfeld geführt werden muss. Dadurch wird ein vergleichsweise kleiner Zugang zum Operationsfeld ermöglicht. Die Verbindung zwischen dem Instrument bzw. der Manipulationseinrichtung einerseits und dem Bauteil andererseits kann vor dem eigentlichen chirurgischen Eingriff bereits hergestellt und nach der Implantation aufgelöst werden.

## Patentansprüche

1. Implantierbares Bauteil (1), das einen im Betrieb innerhalb eines menschlichen oder tierischen Körpers Blut führenden Kanal begrenzt und das insbesondere einen Teil einer Blutfördereinrichtung bildet, **gekennzeichnet durch** ein Koppelelement (10, 14, 19, 23, 24, 28, 32, 32', 44, 54, 55, 56, 62, 67, 68), das an einer dem Blut führenden Kanal abgewandten Fläche des Bauteils angeordnet ist und das zur lösbaren mechanischen Ankopplung einer Manipulationseinrichtung (5, 6, 7, 8, 9, 5', 6', 7', 8', 9', 13, 17, 21, 22, 26, 27, 57, 58, 59) eingerichtet ist.

2. Implantierbares Bauteil nach Anspruch 1, **dadurch gekennzeichnet, dass** das Koppelelement (10, 14, 19, 23, 24, 28, 32, 32', 44, 54, 55, 56, 62, 67, 68) zur selbsthaltenden Ankopplung einer Manipulationseinrichtung (5, 6, 7, 8, 9, 5', 6', 7', 8', 9', 13, 17, 21, 22, 26, 27, 57, 58, 59) eingerichtet ist.

3. Implantierbares Bauteil nach Anspruch 1 oder 2, **dadurch gekennzeichnet, dass** das Koppelelement (10, 14, 19, 23, 24, 28, 32, 32', 44, 54, 55, 56, 62, 67, 68) in das Bauteil (1) integriert ist.

4. Implantierbares Bauteil nach Anspruch 1 oder 2, **dadurch gekennzeichnet, dass** das Koppelelement (10, 14, 19, 23, 24, 28, 32, 32', 44) mit dem Bauteil (1) fest verbunden ist.

5. Implantierbares Bauteil nach einem der Ansprüche 1 bis 4, **dadurch gekennzeichnet, dass** das Koppelelement eine mechanische Fangvorrichtung für ein weiteres Koppelelement einer Manipulationseinrichtung aufweist, wobei die Fangvorrichtung insbesondere eine Einführschräge, insbesondere eine konische, pyramidenförmige oder kalottenförmige Form, aufweist.

6. Implantierbares Bauteil nach einem der Ansprüche 1 bis 5, **dadurch gekennzeichnet, dass** in das Bauteil ein mittels bildgebender Verfahren darstellbares Markerelement (70, 71) in Form eines Oberflächenbelages (71) und/oder eines in der Wand des Bauteils angeordneten Markerkörpers (70) integriert ist.

7. Manipulationseinrichtung (5, 6, 7, 8, 9, 5', 6', 7', 8', 9', 13, 17, 21, 22, 26, 27) zur Handhabung eines in den Körper eines Patienten implantierbaren Bauteils eines Systems zur Blutförderung (1), **gekennzeichnet durch** ein weiteres Koppelelement (4, 4', 11, 12, 19, 20, 25, 29, 31, 33, 36, 37, 45, 57, 58, 59, 63, 64), das zur mechanischen Ankopplung an ein implantierbares Bauteil (1) eingerichtet ist.

8. Manipulationseinrichtung nach Anspruch 7, **dadurch gekennzeichnet, dass** das Koppelelement zur selbsthaltenden Ankopplung einer Manipulationseinrichtung eingerichtet ist.

9. Manipulationseinrichtung nach Anspruch 8, **dadurch gekennzeichnet, dass** als weiteres Koppelelement (57, 58, 59) ein Magnet oder ein ferromagnetischer Körper vorgesehen ist, insbesondere ein schaltbarer Elektromagnet.

10. Koppeleinrichtung zur mechanischen Kopplung eines in einen Patientenkörper implantierbaren Bauteils eines Systems zur Blutförderung (1), einerseits mit einer Manipulationseinrichtung (5, 6, 7, 8, 9, 5', 6', 7', 8', 9', 13, 17, 21, 22, 26, 27), andererseits
**gekennzeichnet durch** ein erstes, dem implantierbaren Bauteil zugeordnetes Koppelelement (10, 14, 19, 23, 24, 28, 32, 32', 44, 54, 55, 56) und ein zweites, der Manipulationseinrichtung zugeordnetes Koppelelement (4, 4', 11, 12, 19, 20, 25, 29, 31, 33, 45, 57, 58, 59), wobei das erste und das zweite Koppelelement kraftschlüssig und/oder formschlüssig und/oder stoffschlüssig oder **durch** magnetische Kräfte miteinander verbindbar sind.

11. Koppeleinrichtung nach Anspruch 10, **dadurch gekennzeichnet, dass** das erste und das zweite Koppelelement derart miteinander verbindbar eingerichtet sind, dass die Kopplung der Koppelelemente selbsthaltend ausgebildet ist.

12. Koppeleinrichtung nach Anspruch 10 oder 11, **dadurch gekennzeichnet, dass** das erste und das zweite Koppelelement derart miteinander verbunden sind, dass eine stoffschlüssige Verbindung zwischen dem ersten Koppelelement und dem zweiten Koppelelement durch Brechen, Zerschneiden oder Änderung der mechanischen Stoffeigenschaften wenigstens eines Teils der Koppeleinrichtung trennbar ist.

13. System zur Blutförderung mit wenigstens einem implantierbaren Bauteil (1) und mit wenigstens einer Manipulationseinrichtung (5, 6, 7, 8, 9, 5', 6', 7', 8', 9', 13, 17, 21, 22, 26, 27), **dadurch gekennzeichnet, dass** das System wenigstens eine Koppeleinrichtung gemäß einem der Ansprüche 10 bis 12 aufweist.

14. Verfahren zum Handhaben eines implantierbaren Bauteils eines Systems zur Blutförderung (1) mit einer Manipulationseinrichtung (5, 6, 7, 8, 9, 5', 6', 7', 8', 9', 13, 17, 21, 22, 26, 27), **dadurch gekennzeichnet, dass** das Bauteil (1) vor dem Einbringen in den Patientenkörper mittels einer selbsthaltenden, lösbaren Koppeleinrichtung mechanisch mit der Manipulationseinrichtung verbunden wird.

15. Verfahren nach Anspruch 14, **dadurch gekennzeichnet, dass** das Bauteil und die Manipulationseinrichtung durch magnetische Kräfte zusammengehalten werden, insbesondere unter Verwendung eines strombeaufschlagten Elektromagneten.

16. Verfahren zum Trennen eines implantierbaren Bauteils (1) von einer Manipulationseinrichtung (5, 6, 7, 8, 9, 5', 6', 7', 8', 9', 13, 17, 21, 22, 26, 27), wobei das Bauteil und die Manipulationseinrichtung mittels einer selbsthaltenden Koppeleinrichtung mechanisch miteinander verbunden sind, **dadurch gekennzeichnet, dass** die Koppeleinrichtung mechanisch gebrochen oder auf andere zerstörende Weise getrennt wird.

17. Verfahren zum Trennen eines implantierbaren Bauteils (1) von einer Manipulationseinrichtung gemäß Anspruch 16, **dadurch gekennzeichnet, dass** ein Teil der Kopplungseinrichtung vor der Trennung mechanisch geschwächt wird.

18. Verfahren zum Trennen eines implantierbaren Bauteils von einer Manipulationseinrichtung gemäß Anspruch 16, **dadurch gekennzeichnet, dass** die Elemente der Kopplungseinrichtung durch eine Betätigung der Manipulationseinrichtung voneinander getrennt werden, die von einer Verschiebung der Manipulationseinrichtung gegenüber dem Bauteil verschieden ist.
